# FASCICULE DE BREVET EUROPEEN

(11) **EP 0 485 583 B1**
(45) Date de publication et mention de la délivrance du brevet: **07.09.1994**
(21) Numéro de dépôt: 91911121.1
(22) Date de dépôt: 06.06.1991
(51) Int. Cl.: A61K 31/47, C07D 217/20, A61K 31/435

(54) **UTILISATION DE DERIVES DE LA TETRAHYDRO ISOQUINOLEINE POUR LA PREPARATION DE MEDICAMENTS ANTI-TUMORAUX**
VERWENDUNG VON TETRAHYDRO-ISOQUINOLINE-DERIVATE ZUR HERSTELLUNG EINES ARZNEIMITTELS MIT ANTITUMORALER WIRKUNG
USE OF TETRAHYDROISOQUINOLINE DERIVATIVES FOR PREPARING ANTITUMORAL DRUGS

(30) Priorité: 08.06.1990 FR 9007137
(43) Date de publication de la demande: 20.05.1992
(73) Titulaire: ROUSSEL-UCLAF, 75007 Paris (FR)
(72) Inventeur: FRECHET, Daniel, F-75015 Paris (FR); MARCHANDEAU, Christian, F-77410 Annet-s/-Marne (FR); CZECH, Jörg, D-3550 Marburg (DE); SEDLACEK, Hans, Harald, D-3550 Marburg (DE)
(74) Mandataire: Bourgouin, André
(86) Numéro de dépôt international: FR9100448
(87) Numéro de publication internationale: WO9118604

(56) Documents cités:
- BIOCHEMICAL PHARMACOLOGY, Vol. 25, No. 9, 1976, GB, pages 1013-1020; L.R.MEYERSON ET COLL.: 'Neroamine derived alkaloids: Substrate.preferred inhibitors of rat brain monoamine oxidase in vitro' see abstract; figures 1,2
- ANNALES PHARMACEUTIQUES FRANCAISES, Vol. 36, No. 9-10, 1978, PARIS FR, pages 401-408; J.-C. GAIGNAULT ET COLL : 'indolylméthyl-1 tétrahydro-1,2,3,4-isoquinoléines et benzyl-1 tétrahydro-2,3,4,9/1H/bétacarbolines formées à partir de la tryptamine et des ses dérivés' see the whole document
- EUROPEAN JOURNAL OF MEDICINAL CHEMISTRY CHIMICA THERAPEUTICA; Vol. 10, No. 1, February 1975, CHATENAY-MALABRY FR, pages 19-28; E. PRUDHOMMEAUX ET COLL.: 'Nouveaux analogues structuraux de la papavérine : benzyl-3 diméthoxy-6,7 isoquinoléines di- et tétra-hydrogénées', see the whole document
- INDUSTRIAL&ENGINEERING CHEMISTRY RESEARCH, Vol. 28, No. 2, 1989, WASHINGTON, US, pages 221-224; C.T. GORALSKI ET COLL.: 'Isoquinoline Alkaloids. 1. An efficient preparation of d,1-laudanosoline Hydrobromide', see page 222, see the whole document
- PATENT ABSTRACTS OF JAPAN, Vol. 013, No. 399 (C-632) 5 September 1989 & JP A-01 143833
- PATENT ABSTRACTS OF JAPAN, Vol. 013, No. 562 (C-665)(3910) 13 December 1989 &JP A-01 233221

## Description

L'invention concerne l'utilisation de dérivés de la tétrahydro isoquinoléïne pour la préparation d'un médicament anti-tumoral et notamment d'un médicament anti-cancéreux, l'application à titre de médicaments de dérivés de la tétrahydro isoquinoléïne et des produits dérivés de cette structure.

L'expression d'oncogènes dans une cellule de mammifère conduit la transformation de types de cellules normales en cellules cancéreuses. Ladite transformation est provoquée par l'infection d'une cellule par un retrovirus. On peut citer comme exemple bien connu l'infection des poules par le virus de Rous qui conduit à l'apparition d'un cancer. L'oncogène correspondant qui est responsable de la transformation maligne a été dénommé gène "SRC" (J.S. Grugge RL Erikson, Nature 269, 346-348 (1977).

Beaucoup des oncogènes connus jusqu'alors sont caractérisés par l'expression d'une protéine possédant une activité de kinase. Ces enzymes catalysent le transfert du groupe phosphate terminal de l'ATP sur un aminoacide. Contrairement à beaucoup d'autres protéinekinases qui transfèrent le groupe phosphate sur un reste séryle ou thréonyle, la plupart des kinases oncogènes phosphorylent un reste tyrosyle de la chaîne proteinique. Par ailleurs, il est connu que les produits d'oncogènes à savoir ceux des oncogènes v-mos, v-mil et v-raf possèdent une activité protéinekinase sérine/thréonine spécifique (K. Rölling et al., Nature (London) 312, 558-561 (1984), B. Singh et al., Journal of Virology 60, 1149-1152 (1986).

L'activité tyrosinekinase fait partie intégrante de la fonction de certains récepteurs de facteurs de croissance. De nouveaux résultats montrent que la croissance de beaucoup de tumeurs dépend de la présence de facteurs de croissance tels que l'Epidermal Growth Factor (EGF) le "Transforming Growth Factor Alpha (TGFAlpha) ou le "Platelet Derived Growth Factor (PDGF) (A.S. Goustin, G. D. Shipley, H.L. Moses, Cancer Research 46, 1015-1029 (1986). Comme conséquence de la liaison du facteur de croissance avec son récepteur, la tyrosinekinase qui est un composant propre du récepteur du facteur de croissance, est stimulée.

On peut donc s'attendre à ce qu'un inhibiteur de la tyrosinekinase et également de la sérine/thréoninekinase puisse inhiber la croissance et la prolifération tumorales et puisse être utilisés dans la thérapie anti-tumorale.

Or il vient d'être découvert de façon surprenante que les produits de formule (I) telle que définie ci-après sont des inhibiteurs de kinases oncogènes telles que la tyrosinekinase, la sérine/thréoninekinase et la tyrosinekinase du récepteur du facteur de croissance et sont donc utilisables pour le traitement de maladies tumorales. Les produits de formule (I) qui présentent des propriétés anti-prolifératives, anti-oncotiques et carcinostatiques peuvent en particulier, être utilisés pour inhiber la croissance et la prolifération tumorales et dans la thérapie tumorale.

L'application de benzylisoquinoléïnes comme produits anti-cancéreux a été décrite dans les demandes japonaises J01143833 et J01233221 et dans la demande WO 90/02119.

La présente invention a pour objet l'utilisation des produits de formule générale (I) : 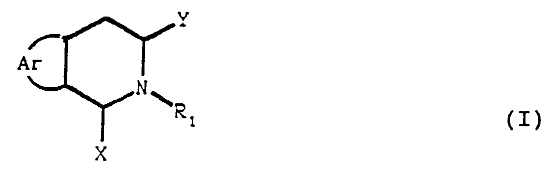
dans laquelle :
X représente un atome d'hydrogène et Y représente un radical : 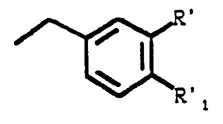
dans lequel R' et R'₁ sont tels que :
ou bien ils sont identiques et représentent chacun un atome d'hydrogène ou un radical hydroxy,
ou bien ils sont identiques ou différents et représentent chacun un radical alkoxy ayant de 1 à 4 atomes de carbone, de préférence méthoxy et Ar représente un radical : 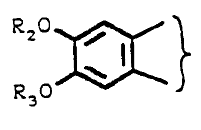
dans lequel R₂ et R₃ identiques ou différents représentent un atome d'hydrogène ou un radical alkyle ayant de 1 à 4 atomes de carbone, de préférence méthyle,
et R₁ représente un atome d'hydrogène ou un radical méthyle, ainsi que leurs sels d'addition avec les acides minéraux ou organiques pour la préparation d'un médicament anti-tumoral.

Les produits qui possèdent un carbone asymétrique peuvent bien entendu se trouver sous forme racémique ou optiquement active. Parmi les valeurs de R' et R'₁, on peut citer outre les valeurs hydrogène et hydroxy les valeurs méthoxy, éthoxy, propyloxy, isopropyloxy, butyloxy, isobutyloxy, tert-butyloxy.

Parmi les valeurs de R₂ et R₃, on peut citer outre la valeur hydrogène, les radicaux suivants :
- méthyle, éthyle, propyle, isopropyle, butyle, isobutyle, sec-butyle, tert-butyle.

Les sels d'addition avec les acides minéraux ou organiques peuvent être, par exemple, les sels formés avec les acides chlorhydrique, bromhydrique, nitrique, sulfurique, phosphorique, acétique, formique, propionique, malonique, maléique, fumarique, succinique, tartrique, citrique, oxalique, glyoxylique, aspartique, alcanesulfoniques tels que les acides méthane ou éthanesulfoniques, arènesulfoniques, tels que les acides benzène ou paratoluène sulfoniques et arylcarboxyliques, tels que benzoïques. Les sels préférés sont les chlorhydrates, bromhydrates et acétates.

Les médicaments objets de la présente invention peuvent se présenter sous la forme de compositions pharmaceutiques destinées à l'administration par voie digestive, parentérale ou locale. Ils peuvent être prescrits sous forme de comprimés simples ou dragéifiés, de gélules, de granulés, de suppositoires, de préparations injectables, de pommades, de crèmes, de gels, lesquels sont préparés selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie varie en fonction de l'affection à traiter et de la voie d'administration, elle peut varier par exemple de 10 à 500 mg par jour chez l'adulte par voie orale. Préférentiellement la dose utilisable peut être de 50 à 250 mg par jour par voie orale.

Parmi les produits ci-dessus, on préfère l'utilisation du 1-[(3,4-dihydroxy phényl) méthyl] 1,2,3,4-tétrahydro isoquinoléïne 6,7-diol pour la préparation d'un médicament anti-tumoral.

L'invention a également pour objet, à titre de médicament le 3-phénylméthyl 1,2,3,4-tétrahydro isoquinoléïne 6,7-diol et le 3-[(3,4-dihydroxyphényl) méthyl] 1,2,3,4-tétrahydro isoquinoléine 6,7-diol ainsi que leurs sels avec les acides minéraux ou organiques pharmacologiquement acceptables.

L'invention a également pour objet les compositions pharmaceutiques contenant à titre de principe actif le médicament tel que défini ci-dessus.

Les compositions pharmaceutiques objet de l'invention peuvent être préparées comme indiqué ci-dessus.

L'invention a également pour objet le 3-phénylméthyl 1,2,3,4-tétrahydro isoquinoléïne 6,7-diol et le 3-[(3,4-dihydroxyphényl) méthyl] 1,2,3,4-tétrahydro isoquinoléine 6,7-diol ainsi que leurs sels avec les acides minéraux ou organiques.

Les produits de formule (I) dans laquelle X représente un atome d'hydrogène et Y représente un radical 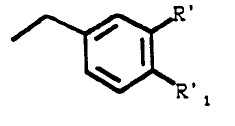
dans lequel R' et R'₁ sont tels que :
ou bien ils sont identiques et représentent chacun un atome d'hydrogène ou un radical hydroxy,
ou bien ils sont identiques ou différents et représentent chacun un radical alkoxy ayant de 1 à 4 atomes de carbone, de préférence méthoxy et Ar représente un radical 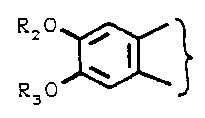
dans lequel R₂ et R₃ ont la signification précédente et R', R'₁ et R₁ ont également la signification précédente, sont des produits connus pour partie. Les produits dans lequels R₁ représente un atome d'hydrogène et R₂ et R₃ représentent chacun un atome d'hydrogène et R' et R'₁ identiques, représentent chacun un atome d'hydrogène ou un radical hydroxy sont des produits nouveaux.

L'invention a donc également pour objet un procédé de préparation du 3-phénylméthyl 1,2,3,4-tétrahydro isoquinoléïne 6,7-diol et du 3-[(3,4-dihydroxyphényl) méthyl] 1,2,3,4-tétrahydro isoquinoléine 6,7-diol et de leurs sels, caractérisé en ce que l'on fait agir un agent d'hydrolyse sur un produit de formule (II) : 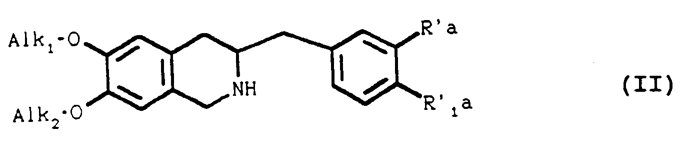
ou un sel de ce produit, dans laquelle Alk₁ et Alk₂ identiques ou différents représentent un radical alkyle ayant de 1 à 4 atomes de carbone et R'ₐ et R'₁ₐ représentent :
ou bien chacun un atome d'hydrogène,
ou bien ils sont identiques ou différents et représentent chacun un radical alkoxy ayant de 1 à 4 atomes de carbone, pour obtenir le produit recherché sous forme de base libre et salifie éventuellement ce produit par un acide minéral ou organique.

Dans un mode préférentiel d'exécution du procédé ci-dessus,
- l'agent d'hydrolyse que l'on fait agir sur le produit de formule (II) ou un sel de ce produit de formule (II) tel que le chlorhydrate est l'acide bromhydrique au reflux. On obtient dans ce cas directement le bromhydrate du produit recherché. Ce produit peut être transformé en base libre à l'aide d'une base telle que la soude, la potasse ou le carbonate acide de sodium.

On utilise comme produit de départ de préférence, le produit de formule (II) dans laquelle Alk₁ et Alk₂ représentent chacun un radical méthyle et R' et R'₁ₐ représentent chacun un atome d'hydrogène ou un radical méthoxy.

Les autres valeurs de Alk₁ et Alk₂ peuvent être choisies parmi les valeurs alkyle indiquées ci-dessus pour R₁ et R₃. Les autres valeurs de R'ₐ et R'₁ₐ peuvent être choisies parmi les valeurs indiquées ci-dessus pour R' et R'₁.

La salification éventuelle du produit recherché est effectuée selon les méthodes usuelles par action de l'acide correspondant.

Certains produits de formule (II) sont connus et notamment le produit de formule (II) dans lequel Alk₁ et Alk₂ représentent chacun un radical méthyle, et R'ₐ et R'₁ₐ représentent chacun un atome d'hydrogène, est décrit dans la publication Eur. J. Med. Chem. Chimica Therapeutica Jan-Fev 1975-10 n° 1 p. 19-28. Les autres produits de ce type peuvent être préparés par les mêmes méthodes.

Les produits de formule (II') correspondant aux produits de formule (II) dans laquelle R'ₐ et R'₁ₐ identiques ou différents représentent chacun un radical alkoxy ayant de 1 à 4 atomes de carbone, peuvent être préparés comme suit :

On fait réagir un produit de formule (III) : 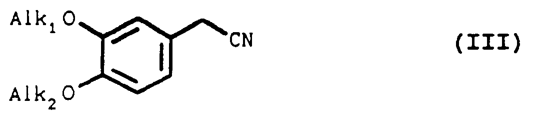
dans laquelle Alk₁ et Alk₂ ont la signification indiquée ci-dessus, avec un produit de formule (IV) en présence d'une base telle que l'éthylate de sodium : 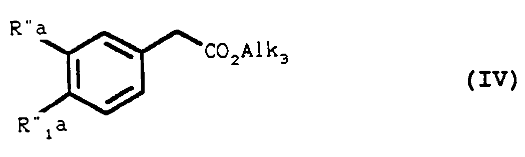
dans laquelle R''ₐ et R''₁ₐ identiques ou différents représentent un radical alkoxy ayant de 1 à 4 atomes de carbone, de préférence méthoxy et Alk₃ représente un radical alkyle ayant de 1 à 4 atomes de carbone, de préférence éthyle, pour obtenir un produit de formule (V) : 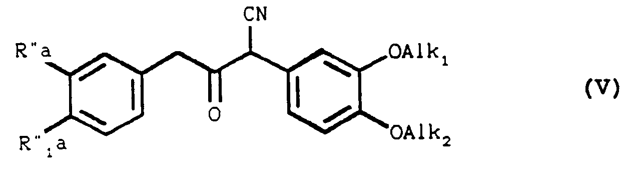
que l'on soumet à une hydrolyse acide, de préférence en présence d'acide sulfurique dilué pour obtenir un produit de formule (VI) : 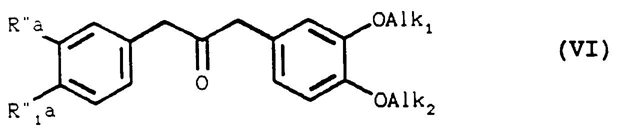
que l'on soumet à l'action de l'hydroxylamine ou d'un sel d'hydroxylamine tel que le chlorhydrate pour obtenir un produit de formule (VII) : 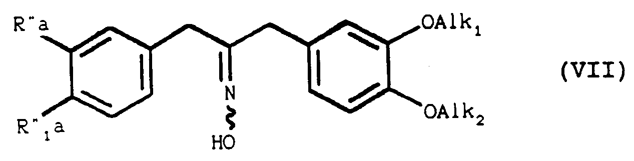
que l'on transforme, par exemple en présence de sodium dans l'éthanol en produit de formule (VIII) : 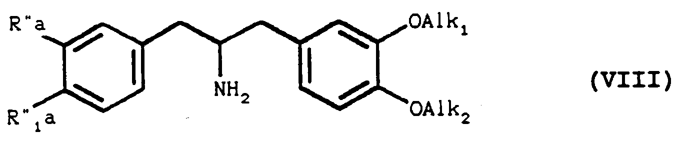
que l'on traite par l'anhydride formique pour obtenir un produit de formule (II') attendu : 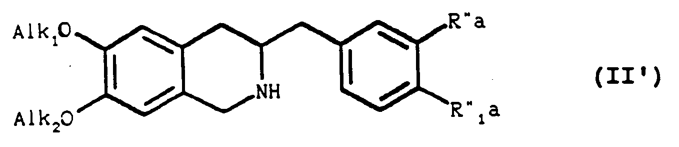
Les produits de formule (I) dans lesquels R₁ représente un radical méthyle peuvent être préparés à partir des produits de formule (II) par méthylation selon les conditions usuelles par exemple à l'aide de sulfate de méthyle ou d'un halogénure de méthyle tel que l'iodure de méthyle.

### EXEMPLE 1 : Bromhydrate du 3-phénylméthyl 1,2,3,4-tétrahydro, isoquinoléïne 6,7-diol

Une solution de 3,75 g de chlorhydrate du 6,7-diméthoxy 3-phénylméthyl 1,2,3,4-tétrahydro isoquinoléïne décrit dans la publication Eur. J. Med. Chem. Chemica Thérapeutica Jan-Fév. 1975-10 n° 1 p. 19-28 dans 37 cm³ d'acide bromhydrique à 48 %, est portée au reflux pendant une heure puis glacée, essorée, lavée à l'eau glacée puis à l'éther et séchée sous pression réduite. On obtient 3,4 g de produit F = 255°C.

Ce produit est purifié comme suit :
On dissout ce produit dans 100 cm³ d'éthanol au reflux, filtre à chaud, concentre à 30 cm³, amorce, laisse à 4-5°C essore, lave deux fois à l'éthanol glacé puis sèche à 60°C sous pression réduite. On obtient 1,8 g de produit F = 255°C.

On amène à sec les liqueurs mères, dissout le résidu dans 10 cm³ de méthanol, ajoute de l'acétate d'éthyle, concentre jusqu'à cristallisation, glace, essore, lave à l'acétate d'éthyle puis sèche. On obtient 1,5 g de produit F = 250°C.

Ces deux jets (3,3 g) sont dissous dans 20 cm³ de méthanol au reflux. On filtre à chaud, ajoute de l'acétate d'éthyle concentre jusqu'à cristallisation, laisse une nuit à 4-5°C, essore, lave à l'acétate d'éthyle, glace puis sèche une heure à 100°C. On obtient 2,1 g de produit attendu F ≃ 256°C.

### Infra-rouge (nujol)

Aromatiques : 1631 - 1612 - 1582 (forte) - 1529 (forte) - 1499 cm⁻¹

### Ultra-violet

a) dans EtOH
   infl 226 nm E'₁ = 192
   infl 259 nm E'₁ = 17
   infl 264 nm E'₁ = 25.
   max. 287-288 nm E'₁ = 118 epsilon = 3950
b) dans EtOH, NaOH 0,1 N
   max. 275 nm
   max. 330 nm
   infl. 380 nm.

### EXEMPLE 2 : compositions pharmaceutiques

On a préparé des gélules répondant à la formule :

| | |
|---|---|
| - produit de l'exemple 1 | 50 mg |
| - excipient q.s.p. une gélule terminée à | 400 mg |

### Test pharmacologique :

Dans le test suivant les produits sont dénommés comme suit :
- le produit A est le 1-[(3,4-dihydroxy phényl) méthyl] 1,2,3,4-tétrahydro isoquinoléïne 6,7-diol,
- le produit B est le 1,2-benzènediol 4-(2,3,4,9-tétrahydro 6-hydroxy-1H-pyrido[3,4-b]indol 1-yl) méthyl.

Mesure de l'inhibition de la tyrosinekinase du récepteur de l'EGF (Epidermal Growth Factor).

On utilise des membranes de cellules A431 (ATCC CRL 1555) comme source de récepteurs de l'EGF. Cette lignée cellulaire exprime à sa surface un grand nombre de récepteur de l'EGF qui possèdent une activité tyrosinekinase.

Ces cellules sont préincubées avec ou sans EGF (1000 nM) pendant 15 minutes et ajoutées au tampon HEPES (acide N-2-hydroxyéthyl-piperazine N'-2-éthanesulfonique) correspondant (avec ou sans EGP) et contenant des ions Mg²⁺ (10 mM) et Mn²⁺ (2 mM) 0,2 % de triton X-100, de l'orthovanadate de sodium (20 micromoles) et l'inhibiteur.

Des échantillons contenant ou ne contenant pas le substrat poly(Glu, Ala, Tyr 6 : 3 : 1) sont préparés. On initie la réaction par addition de [gamma³²P] ATP (32 micromoles). Après incubation pendant 15 minutes à 30°C les échantillons sont précipités à l'aide d'acide chloroacétique à 10 %, filtrés sur membrane Millipore ® et le ³²P incorporé est mesuré avec un compteur de scintillation liquide.

Les résultats sont exprimés en CI₅₀, c'est-à-dire la concentration de substance qu'inhibe 50 % de l'activité de l'enzyme, déterminée par une série de dilutions commençant à 51 microgrammes/ml dans les échantillons contenant le substrat et l'EGF.

On obtient les résultats suivants :
Produit A : 1,1 microgrammes/ml.
Produit B : 2,5 microgrammes/ml.
Produit de l'exemple 1 : 3,4 microgrammes/ml.

## Revendications

1. Utilisation des produits de formule générale (I) : 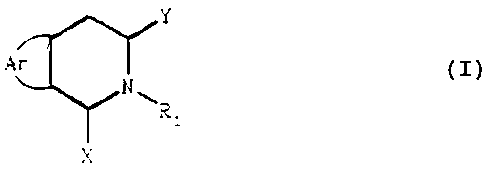 dans laquelle :
X représente un atome d'hydrogène et Y représente un radical : 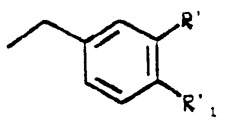 dans lequel R' et R'₁ sont tels que :
ou bien ils sont identiques et représentent chacun un atome d'hydrogène ou un radical hydroxy,
ou bien ils sont identiques ou différents et représentent chacun un radical alkoxy ayant de 1 à 4 atomes de carbone, de préférence méthoxy et Ar représente un radical : 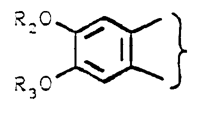 dans lequel R₂ et R₃ identiques ou différents représentent un atome d'hydrogène ou un radical alkyle ayant de 1 à 4 atomes de carbone, de préférence méthyle,
et R₁ représente un atome d'hydrogène ou un radical méthyle, ainsi que leurs sels d'addition avec les acides minéraux ou organiques pour la préparation d'un médicament anti-tumoral.

2. A titre de médicament le 3-phénylméthyl 1,2,3,4-tétrahydro isoquinoléïne 6,7-diol et le 3-[(3,4-dihydroxyphényl) méthyl] 1,2,3,4-tétrahydro isoquinoléine 6,7-diol ainsi que leurs sels avec les acides minéraux ou organiques pharmacologiquement acceptables.

3. Les compositions pharmaceutiques contenant, comme principe actif l'un au moins des médicaments selon la revendication 2.

4. Le 3-phénylméthyl 1,2,3,4-tétrahydro isoquinoléïne 6,7-diol et le 3-[(3,4-dihydroxyphényl) méthyl] 1,2,3,4-tétrahydro isoquinoléine 6,7-diol et leurs sels avec les acides minéraux ou organiques.

5. Procédé de préparation du produit décrit à la revendication 4 et de leurs sels, caractérisé en ce que l'on fait agir un agent d'hydrolyse sur un produit de formule (II) : 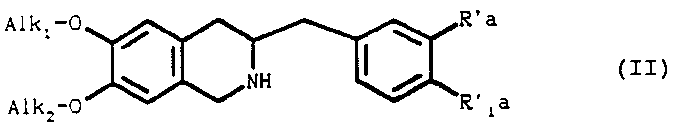 ou un sel de ce produit, dans laquelle Alk₁ et Alk₂ identiques ou différents représentent un radical alkyle ayant de 1 à 4 atomes de carbone et R'ₐ et R'₁ₐ représentent :
ou bien chacun un atome d'hydrogène,
ou bien ils sont identiques ou différents et représentent chacun un radical alkoxy ayant de 1 à 4 atomes de carbone, pour obtenir le produit recherché sous forme de base libre et salifie éventuellement ce produit par un acide minéral ou organique.

## Claims

1. Use of the products of general formula (I): 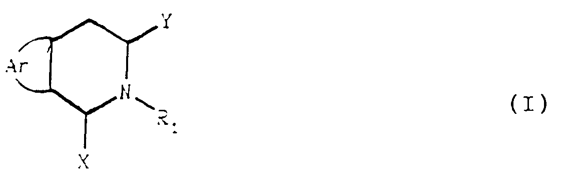 in which:
X represents a hydrogen atom and Y represents a radical: 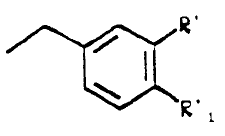 in which R' and R'₁ are such that:
either they are identical and each represent a hydrogen atom or a hydroxy radical,
or they are identical or different and each represent an alkoxy radical having 1 to 4 carbon atoms, preferably methoxy and Ar represents a radical: 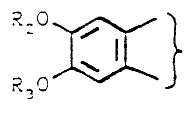 in which R₂ and R₃ identical or different represent a hydrogen atom or an alkyl radical having 1 to 4 carbon atoms, preferably methyl,
and R₁ represents a hydrogen atom or a methyl radical as well as their addition salts with mineral or organic acids for the preparation of an anti-tumoral medicament.

2. As a medicament 3-phenylmethyl 1,2,3,4-tetrahydro isoquinoline 6,7-diol and 3-[(3,4-dihydroxyphenyl) methyl] 1,2,3,4-tetrahydro isoquinoline 6,7-diol as well as their salts with pharmaceutically acceptable mineral or organic acids.

3. The pharmaceutical compositions containing at least one of the medicaments according to claim 2 as active ingredient.

4. 3-phenylmethyl 1,2,3,4-tetrahydro isoquinoline 6,7-diol and 3-[(3,4-dihydroxyphenyl) methyl] 1,2,3,4-tetrahydro isoquinoline 6,7-diol and their salts with mineral or organic acids.

5. Preparation process for the product described in claim 4 and their salts, characterized in that a hydrolysis agent is reacted on a product of formula (II): 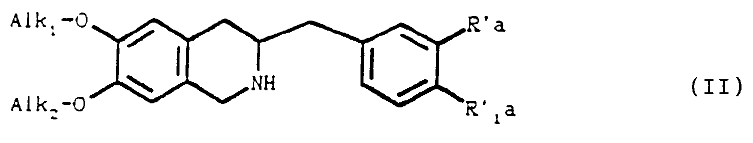 or a salt of this product, in which Alk₁ and Alk₂, identical or different, represent an alkyl radical having 1 to 4 carbon atoms and R'ₐ and R'₁ₐ represent:
either each of them a hydrogen atom,
or they are identical or different and each represent each an alkoxy radical having 1 to 4 carbon atoms, in order to obtain the desired product in the form of the free base and this product is optionally salified by a mineral or organic acid.

## Patentansprüche

1. Verwendung der Produkte der allgemeinen Formel (I) 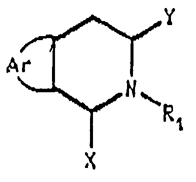 in der:
X ein Wasserstoffatom darstellt und Y einen Rest 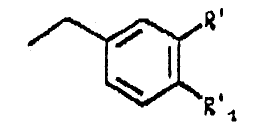 bedeutet, worin R' und R'₁ sind:
entweder sind sie gleich und stellen jeweils ein Wasserstoffatom oder einen Hydroxyrest dar,
oder sie sind gleich oder verschieden und stellen jeweils einen Alkoxyrest mit 1 bis 4 Kohlenstoffatomen dar, vorzugsweise Methoxy, und Ar bedeutet einen Rest 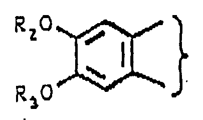 worin R₂ und R₃, gleich oder verschieden, ein Wasserstoffatom oder einen Alkylrest mit 1 bis 4 Kohlenstoffatomen, vorzugsweise Methyl, darstellen,
und R₁ ist ein Wasserstoffatom oder ein Methylrest, sowie ihrer Additionssalze mit Mineralsäuren oder organischen Säuren zur Herstellung eines Arzneimittels mit antitumoraler Wirkung.

2. Als Arzneimittel das 3-Phenylmethyl-1,2,3,4-tetrahydro-isochinolin-6,7-diol und das 3-[(3,4-Dihydroxyphenyl)-methyl]-1,2,3,4-tetrahydro-isochinolin-6,7-diol sowie ihre Salze mit pharmakologisch akzeptablen Mineralsäuren oder organischen Säuren.

3. Pharmazeutische Zusammensetzungen, die als Wirkstoff mindestens eines der Arzneimittel nach Anspruch 2 enthalten.

4. 3-Phenylmethyl-1,2,3,4-tetrahydro-isochinolin-6,7-diol und 3-[(3,4-Dihydroxyphenyl)-methyl]-1,2,3,4-tetrahydro-isochinolin-6,7-diol und ihre Salze mit Mineralsäuren oder organischen Säuren.

5. Verfahren zur Herstellung des in Anspruch 4 beschriebenen Produktes und seiner Salze, dadurch gekennzeichnet, daß man ein Hydrolysierungsmittel mit einem Produkt der Formel (II) 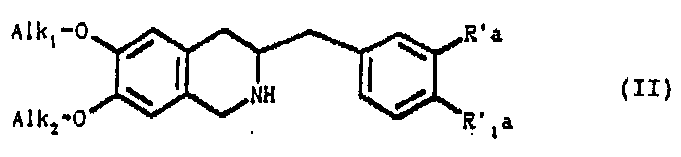 oder mit einem Salz dieses Produktes zur Reaktion bringt, worin Alk₁ und Alk₂, gleich oder verschieden, einen Alkylrest mit 1 bis 4 Kohlenstoffatomen darstellen und R'ₐ und R'₁ₐ bedeuten:
entweder jeweils ein Wasserstoffatom,
oder sie sind gleich oder verschieden und stellen jeweils einen Alkoxyrest mit 1 bis 4 Kohlenstoffatomen dar,
um das gesuchte Produkt in Form der freien Base und gegebenenfalls durch eine Mineralsäure oder organische Säure in Salz überführt zu erhalten.
